# EUROPEAN PATENT SPECIFICATION

(11) **EP 3 560 913 B1**
(45) Date of publication and mention of the grant of the patent: **29.07.2026**
(21) Application number: 18169142.9
(22) Date of filing: 25.04.2018
(51) Int. Cl.: C07D 257/04

(54) **PROCESS FOR THE PRODUCTION OF TETRAZOLINONES**
VERFAHREN ZUR HERSTELLUNG VON TETRAZOLINONEN
PROCÉDÉ DE PRODUCTION DE TÉTRAZOLINONES

(43) Date of publication of application: 30.10.2019
(73) Proprietor: Dynamit Nobel GmbH Explosivstoff- und Systemtechnik, 51377 Leverkusen (DE)
(72) Inventor: Haller, Jan, 51379 Leverkusen (DE)
(74) Representative: Bandpay & Greuter

(56) References cited:
- EP-A1- 0 711 761
- US-A- 5 530 135
- TAREK A. SALAMA ET AL: "Silicon-mediated Direct Conversion of Acyl Chlorides to Carbamoyl Azides or/and Tetrazolinones under Mild Conditions", CHEMISTRY LETTERS, vol. 40, no. 10, 5 October 2011 (2011-10-05), JAPAN, pages 1149 - 1151, XP055487745, ISSN: 0366-7022, DOI: 10.1246/cl.2011.1149

## Description

### TECHNICAL FIELD

The present invention relates to a process for the production of tetrazolinones.

### TECHNICAL BACKGROUND

Agricultural crops, damaged or destroyed by pests, represent an important issue worldwide. In fact, the costs of such damage exceed billions annually due to decreased crop yields, reduced crop quality and increased harvesting costs. Therefore, a strong demand exists for the control of pests in agricultural crops by safe and effective means, which would have no harmful effects to mammals and other living organisms.

It has been disclosed in documents US 4956469, US 5003075 and US 5019152 that tetrazolinone derivatives exhibit pesticidal properties and can be useful as plant growth regulators.

Furthermore, it has been found that tetrazolinone derivatives exhibit interesting properties which can be useful in the fields of medicinal chemistry or electronics. For instance, document US 5705653 mentions that tetrazolinone derivatives can be used as β₃ agonists for the treatment of obesity and diabetes, while document US 2017/0200619 discloses the use of tetrazolinone derivatives for the removal of anti-reflective material and/or post-etch residue from micro electronic devices.

Documents US 4839349, US 5530135, WO 2005/112941, WO 2013/162072 and US 2014/364414 disclose the preparation of tetrazolinone derivatives by reacting an isocyanate compound with sodium azide in the presence of aluminum chloride in tetrahydrofuran (THF) or dimethylformamide (DMF). However, aluminum chloride is a very hygroscopic solid that reacts violently with water. Its addition to DMF, which is one of the preferred reaction solvents, provokes heat generation. The hydrochloric acid generated after the reaction of aluminum chloride with water can then react with sodium azide to generate the explosive hydrazoic acid. Furthermore, the aluminum salts resulting from the aqueous work-up tend to cause problems during filtration.

Documents US 5066667, US 5347010, WO 2004/80984, US 2011/130415, *"*Reactions of trimethylsilyl azide with heterocumulenes" (Journal of Organic Chemistry, 1980, 45, 5130), *"*Optically active antifungal azoles. VI. Synthesis and antifungal activity of N-[(1R,2R)-2-(2,4-Difluorophenyl)-2-hydroxy-1-methyl-3-(1H-1,2,4-triazol-1-yl)propyl]-N'-(4-substituted phenyl)-3(2H,4H)-1,2, 4-triazolones and 5(1H,4H)-tetrazolones" (Chemical and Pharmaceutical Bulletin, 1996, 44, 314) and *"*Electronic properties of 1-methyl-4-phenyl-1H-tetrazole-5(4H)-thiones: An experimental and theoretical study" (Journal of Molecular Structure, 2009, 933, 38) disclose the preparation of tetrazolinone derivatives by reacting an isocyanate compound with a stoichiometric amount of trimethylsilyl azide either in benzene or without a solvent. Nevertheless, trimethylsilyl azide is a volatile compound, easily hydrolysable and highly toxic, which must be handled with great care in order to avoid inhalation and intoxication. In addition, trimethylsilyl azide is an expensive reagent and only 40% of its molar mass is actually found in the final product, the rest of the compound creating waste.

There is thus a need for novel methods for the production of tetrazolinones, that overcome the abovementioned issues and offer a safe and efficient route to such compounds.

### SUMMARY OF THE INVENTION

It is a first object of the invention to provide a process for the preparation of a tetrazolinone compound, comprising a step of reacting an isocyanate compound with an azide salt in a solvent and in the presence of a silicon derivative as a catalyst, as defined in claim 1.

According to some embodiments, the azide salt is sodium azide.

According to some embodiments, the solvent is an amide, preferably selected from dimethylformamide, dimethylacetamide, N-methylpyrrolidone and combinations thereof.

According to some embodiments, the silicon derivative is a trialkylsilyl azide, preferably trimethylsilyl azide.

The silicon derivative is prepared by reacting a silyl halide compound with an azide salt.

According to some embodiments, the silyl halide compound is a trialkylsilyl halide.

According to some embodiments, the silyl halide compound is a silyl chloride compound.

According to some embodiments, the silyl halide compound is trimethylsilyl chloride.

The silicon derivative is employed in a molar amount of less than 0.5 molar equivalent and more preferably less than 0.1 molar equivalent, relative to the isocyanate compound.

According to some embodiments, the isocyanate compound is an aryl isocyanate and the tetrazolinone compound is a 1-aryl-tetrazolinone.

According to some embodiments, the step of reacting the isocyanate compound with the azide salt is performed at a temperature of at least 60°C, preferably of at least 80°C, and more preferably of at least 100°C.

According to some embodiments, a tetrazolinone salt is obtained after the step of reacting the isocyanate compound with the azide salt; and the process further comprises an additional step of treating the tetrazolinone salt with an acid to obtain the tetrazolinone compound.

According to some embodiments, the process comprises an intermediate purification step between the step of reacting the isocyanate compound with the azide salt and the additional step of treating the tetrazolinone salt with the acid.

According to some embodiments, the process further comprises a step of treating the tetrazolinone salt with sodium nitrite, before the additional step of treating the tetrazolinone salt with the acid, and preferably after the intermediate purification step.

The present invention makes it possible to address the need expressed above. In particular the invention provides a process for the production of tetrazolinones, that overcome the abovementioned issues and offer a safe and efficient route to such compounds.

This is achieved with the use of a silicon derivative as a catalyst, which is more easily handled and less hazardous than aluminum chloride. The invention also makes it possible to avoid using large quantities of expensive reagents, such as trimethylsilyl azide.

### DESCRIPTION OF EMBODIMENTS

The invention will now be described in more detail without limitation in the following description.

The present invention relates to a process for the preparation of a tetrazolinone compound, comprising a step of reacting an isocyanate compound with an azide salt in a solvent and in the presence of a silicon derivative as a catalyst, as defined in claim 1.

The isocyanate compound used for the preparation of the tetrazolinone compound has the general formula (I):

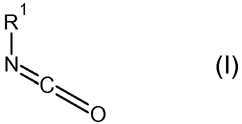

In this formula, R¹ can be any carbon-containing group, such as an aryl group, an aryl-containing group or a non-aryl-containing group.
R¹ can preferably be chosen from:
- C₁-C₁₂ alkyl;
- C₂-C₁₃ alkoxyalkyl;
- C₇-C₉ aralkyl;
- C₃-C₆ cycloalkyl;
- C₃-C₁₂ alkenyl;
- C₃-C₆ cycloalkenyl;
- C₃-C₁₂ alkynyl;
- naphthyl;
- phenyl;
- phenyl substituted with at least one substituent selected from the group consisting of:
   ∘ fluorine;
   ∘ chlorine;
   ∘ bromine;
   ∘ iodine;
   ∘ C₁-C₈ alkyl;
   ∘ C₁-C₈ alkoxy;
   ∘ methylenedioxy;
   ∘ NR²R³ wherein R² and R³ can be the same or different and can be hydrogen or C₁-C₁₂ alkyl;
   ∘ C₁-C₄ alkoxycarbonyl;
   ∘ carboxy;
   ∘ phenoxy;
   ∘ nitro;
   ∘ cyano;
   ∘ trihalomethyl wherein each halo can be fluoro, chloro or bromo (the same or different, preferably the same);
   ∘ trihalomethoxy wherein each halo can be fluoro, chloro or bromo (the same or different, preferably the same);
   ∘ C₁-C₆ alkylthio;
   ∘ C₁-C₆ fluoroalkylthio;
- benzyl;
- benzyl substituted with at least one substituent selected from the group consisting of:
   ∘ fluorine;
   ∘ chlorine;
   ∘ bromine;
   ∘ iodine;
   ∘ C₁-C₈ alkyl;
   ∘ C₁-C₈ alkoxy;
   ∘ methylenedioxy;
   ∘ C₁-C₄ alkoxycarbonyl;
   ∘ phenoxy;
   ∘ nitro;
   ∘ cyano;
   ∘ trihalomethyl wherein each halo can be fluoro, chloro or bromo;
   ∘ trihalomethoxy wherein each halo can be fluoro, chloro or bromo (the same or different, preferably the same);
   ∘ C₁-C₆ alkylthio;
   ∘ C₁-C₆ fluoroalkylthio;
- C₂-C₅ aromatic heterocycles containing at least one heteroatom, wherein the heteroatom can be oxygen, nitrogen or sulfur;
- C₂-C₅ non-aromatic heterocycles containing at least one heteroatom, wherein the heteroatom can be oxygen, nitrogen or sulfur.

The alkyl, alkoxy and alkylthio radicals used as substituents can be straight-chain or branched. For example, such alkyl radicals can include methyl, ethyl, propyl, isopropyl, butyl, i-butyl, sec-butyl, tert-butyl, hexyl, octyl and their isomers. Suitable alkoxy radicals include for example methoxy, ethoxy, isopropoxy, butoxy and their isomers. Alkylthio radicals can be for example methylthio, ethylthio, isopropylthio, propylthio, butylthio, i-butylthio, sec-butylthio and tert-butylthio. Substituents composed of several generic groups such as alkoxyalkyl and fluoroalkylthio contain appropriate combinations of the abovementioned individual substituents.

Cycloalkyl radicals used as substituents can be for example: cyclopropyl, cyclobutyl, cyclopentyl and cyclohexyl.

The alkenyl and alkynyl radicals used as substituents can be straight-chain or branched and contain one or more unsaturated bonds. Double and triple bonds can be present in the same radical. Such alkenyl and alkynyl radicals can be for example: vinyl, allyl, 1-propenyl, isopropenyl, allenyl, butenyls, butadienyls, hexenyls, ethynyl, 1-propynyl, 2-propynyl, butinyls, pentynyls, hexynyls, hexadienyls and 2-pent-en-4-ynyl.

Examples of alkoxycarbonyl radicals are: methoxycarbonyl, ethoxycarbonyl, propoxycarbonyl, isopropoxycarbonyl and butoxycarbonyl.

Examples of aromatic heterocycles are: furan, pyrrole, thiophene, pyridine, pyridazine, pyrimidine, imidazole, pyrazole, thiazole, isoxazole, oxazole, triazole, oxadiazole, thiadiazole and triazine.

Examples of non-aromatic heterocycles are: ethylene sulfide, aziridine, trimethylene oxide, trimethylene sulfide, azetidine, tetrahydrofurane, pyrrolidine, thiolane, pyran, tetrahydropyran, piperidine, thiacyclohexane, dioxane, piperazine and morpholine.

Preferably, R¹ is an aryl group and the isocyanate compound is an aryl isocyanate.

Examples of preferred isocyanate compounds include phenyl isocyanate, 2-chlorophenyl isocyanate, 3-chlorophenyl isocyanate, 4-chlorophenyl isocyanate, 2-bromophenyl isocyanate, 3-bromophenyl isocyanate, 4-bromophenyl isocyanate, 2-methylphenyl isocyanate, 3-methylphenyl isocyanate, and 4-methylphenyl isocyanate.

The azide salt can be notably chosen from sodium azide, potassium azide, lithium azide, or ammonium azide. The ammonium azide can notably be a quaternary ammonium azide salt, such as a tetraalkylammonium azide.

Examples of tetraalkylammonium azides are tetramethylammonium azide, tetraethylammonium azide, and tetrabutylammonium azide.

The azide salt is preferably sodium azide.

The solvent is preferably a polar solvent. Preferably the solvent comprises an amide such as dimethylformamide, dimethylacetamide or N-methylpyrrolidone.

According to some (preferred) embodiments, the solvent used in the process consists of a single compound chosen from the abovementioned compounds.

*By "silicon derivative"* is meant a silicon-containing compound, preferably a compound which contains a single silicon atom.

According to the present invention, the silicon derivative is used as a catalyst, which means that the silicon derivative is substantially not consumed during the step of reacting the isocyanate compound with the azide salt.

The silicon derivative that is used as a catalyst has the general formula (II):

In formula (II), 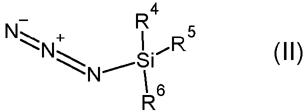 each of R⁴, R⁵ and R⁶ are independently selected from aryl, alkyl, aralkyl and alkoxyalkyl groups.

Each of R⁴, R⁵ and R⁶ can notably be chosen from a methyl group, an ethyl group, an isopropyl group, a tert-butyl group or a phenyl group.

R⁴, R⁵ and R⁶ can be the same or different.

If the substituents R⁴, R⁵ and R⁶ are the same, they can be for example alkyl groups (trialkylsilyl azide), such methyl groups (trimethylsilyl azide), ethyl groups (triethylsilyl azide) or isopropyl groups (triisopropylsilyl azide).

If R⁴, R⁵ and R⁶ are different, R⁴ and R⁵ can be for example a methyl group and R⁶ can be an isopropyl group (isopropyldimethylsilyl azide), or R⁴ and R⁵ can be an ethyl group and R⁶ can be an isopropyl group (diethyliisopropylsilyl azide), or R⁴ and R⁵ can be a methyl group and R⁶ can be a tert-butyl group (tert-butyldimethylsilyl azide), or R⁴ and R⁵ can be a phenyl group and R⁶ can be a tert-butyl group (tert-butyldiphenylsilyl azide).

Preferably, the silicon derivative is trimethylsilyl azide.

In some embodiments, the silicon derivative is prepared prior to or during the step of reacting the isocyanate compound with the azide salt. The preparation may be performed in the same reaction vessel used for reacting the isocyanate compound with the azide salt.

The preparation of the silicon derivative is made by reacting a silyl halide compound with an azide salt. The azide salt used in this preparation step can notably be as defined above. It can be the same azide salt also used for the reaction with the isocyanate compound or a different one. Preferably it is the same. Sodium azide is a preferred salt for preparing the silicon derivative.

According to some embodiments, the silyl halide compound can have the general formula (III):

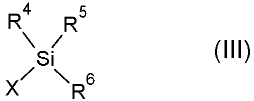

R⁴, R⁵ and R⁶ are as defined above.

X is a halogen, preferably selected from chlorine, bromine and iodine. The silyl halide compound can be chosen from a silyl chloride compound, or a silyl bromide compound, or a silyl iodide compound. Preferably, the silyl halide compound is a silyl chloride compound, and more preferably, the silyl halide compound is a trialkylsilyl chloride, most preferably trimethylsilyl chloride.

According to some embodiments, the silicon derivative can be prepared by the addition of the azide salt to a mixture containing the silyl halide and the solvent.

Alternatively and preferably, the silicon derivative can be prepared by the addition of the silyl halide to a mixture containing the azide salt and the solvent.

The isocyanate compound may be added subsequently or may be present in the above mixture containing the silyl halide and solvent or containing the azide salt and the solvent.

The silicon derivative is present in a molar amount of less than 0.5 equivalent and more preferably less than 0.1 equivalent, relative to the isocyanate compound.

The silicon derivative can for example be employed in a molar amount from 0.4 to 0.5 equivalent, or from 0.3 to 0.4 equivalent, or from 0.2 to 0.3 equivalent, or from 0.1 to 0.2 equivalent, or from 0.075 to 0.1 equivalent, or from 0.05 to 0.075 equivalent, or from 0.025 to 0.05 equivalent, or from 0.01 to 0.025 equivalent, relative to the isocyanate compound.

According to some embodiments, the azide salt can be present in a molar amount of from 0.5 to 5.0 equivalent, relative to the isocyanate compound. Preferably, the azide salt is present in a molar amount of at least 1.0 equivalent, or at least 1.01 equivalent, or at least 1.02 equivalent, or at least 1.03 equivalent, or at least 1.04 equivalent, or at least 1.05 equivalent, relative to the isocyanate compound.

The azide salt can for example be employed in a molar amount from 0.5 to 0.9 equivalent, or from 0.9 to 1.0 equivalent, or from 1.0 to 1.1 equivalent, or from 1.1 to 1.2 equivalent, or from 1.2 to 1.5 equivalent, or from 1.5 to 2.0 equivalent, or from 2.0 to 5.0 equivalent, relative to the isocyanate compound.

The above amounts correspond to the reactants initially put into contact, after the optional step of preparing the silicon derivative.

According to some embodiments, the step of reacting the isocyanate compound with the azide salt can be performed at a temperature of at least 60°C, preferably of at least 80°C, and more preferably of at least 100°C.

The step of reacting the isocyanate compound with the azide salt can notably be performed at a temperature from 60 to 70°C, or from 70 to 80°C, or from 80 to 90°C, or from 90 to 100°C, or from 100 to 110°C, or from 110 to 120°C, or from 120 to 130°C, or from 130 to 140°C, or from 140 to 150°C.

According to some embodiments, the isocyanate compound is added to a reaction mixture comprising the azide salt and/or the silicon derivative. Said addition can in particular be carried out in less than 1 hour, more preferably in less than 50 minutes, and more preferably in less than 45 minutes.

According to some embodiments, after the step of reacting the isocyanate compound with the azide salt, a tetrazolinone salt is obtained.

In particular, the tetrazolinone salt may have the general formula (IV):

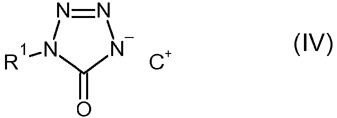

R¹ is as defined above.

C⁺ is a cation coming from the cation present in the azide salt, and it can be notably chosen from sodium, potassium, lithium and ammonium ions. The ammonium ions can notably be quaternary ammonium ions, such as tetraalkylammonium ions.

Examples of tetraalkylammonium ions are tetramethylammonium ions, tetraethylammonium ions, and tetrabutylammonium ions.

Preferably, C⁺ is a sodium ion.

The desired tetrazolinone compound can be the above tetrazolinone salt. In this case, the tetrazolinone salt, which is soluble in the solvent in this step, can be recovered, and optionally washed and/or purified, e.g. by filtration. The aim of the filtration is to remove solid by-products.

Alternatively, the above tetrazolinone salt may be further converted to the desired tetrazolinone compound. Accordingly, the process may further comprise an additional step of treating the tetrazolinone salt with an acid. The acid may for instance directly be added to the reaction mixture obtained from the step of reacting the isocyanate compound with the azide salt. Alternatively, the tetrazolinone salt, which is soluble in the solvent in this step, may be filtered to remove solid by-products, prior to being treated with the acid.

The acid used to convert the tetrazolinone salt to the tetrazolinone compound can notably be used in an amount such that the pH of the medium containing the tetrazolinone salt is reduced to less than 2, or less than 1.5, or more preferably less than 1. Such acid can be either an inorganic or an organic acid. Examples of inorganic acids are: hydrochloric acid, hydrobromic acid, hydroiodic acid, sulfuric acid, phosphoric acid, carbonic acid, and nitric acid. Examples of organic acids are: trifluoroacetic acid, trichloroacetic acid, methanesulfonic acid, ethane sulfonic acid, benzenesulfonic acid, p-toluenesulfonic acid, trifluoromethanesulfonic acid, and formic acid. Combinations of the above acids can also be used. Preferably, the acid used to transform the tetrazolinone salt to the tetrazolinone compound is hydrochloric acid or sulfuric acid.

According to some embodiments, after filtration of the solid by-products and prior to being treated with the acid, the tetrazolinone salt can be treated with a substance such as e.g. sodium nitrite in order to provoke the decomposition of residual azide. The amount of sodium nitrite may be adjusted so as to fully decompose the residual azide.

Sodium nitrite can preferably be used in a molar amount of from 0.01 to 1.0 equivalent, relative to the isocyanate compound. In some embodiments, sodium nitrite is used in a molar amount of less than 1.0 equivalent, or less than 0.9 equivalent, or less than 0.8 equivalent, or less than 0.7 equivalent, or less than 0.6 equivalent, or less than 0.5 equivalent, or less than 0.4 equivalent, or less than 0.3 equivalent, or less than 0.2 equivalent, or less than 0.1 equivalent, or less than 0.05, or less than 0.02 equivalent, or equal to 0.01 equivalent, relative to the isocyanate compound.

The desired tetrazolinone compound can then be recovered, and optionally washed and/or purified, e.g. by filtration.

The desired tetrazolinone can also be used in another reaction for the synthesis of tetrazolinone derivatives, as for example described in US 4839349.

The tetrazolinone compound thus obtained has the general formula (V):

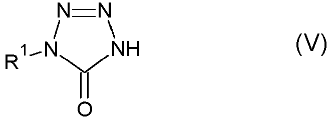

R¹ is as defined above.

Accordingly, the tetrazolinone compounds obtained by the process of the invention are preferably 1-substituted-5(4H)-tetrazolinones.

Preferably, R¹ is an aryl group and the tetrazolinone compound is a 1-aryl-tetrazolinone.

By way of illustration, sodium azide can be used as the azide salt and trimethylsilyl chloride can be used as the silyl halide derivative to form the silicon derivative, which in this case is trimethylsilyl azide. The corresponding reaction scheme is shown below.

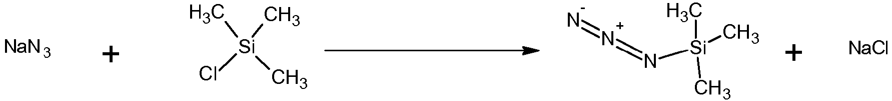

Trimethylsilyl azide can then react with an isocyanate compound having the general formula (I), to give an intermediate trimethylsilyl tetrazolinone which after reaction with sodium azide can lead to the formation of the tetrazolinone sodium salt and trimethylsilyl azide which is thus regenerated, as shown below.

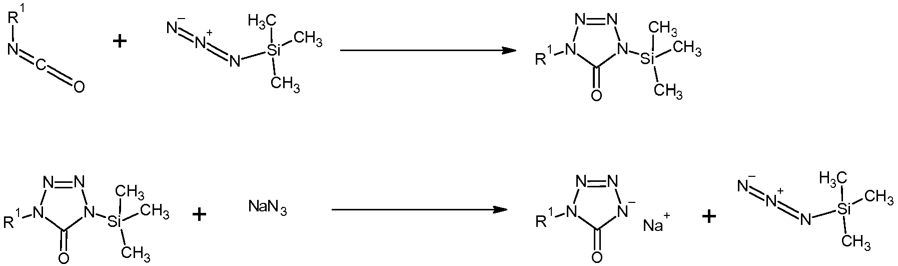

The tetrazolinone salt can either be isolated as is or, alternatively and preferably, the tetrazolinone salt can be treated with an acid (for example hydrochloric acid) to give the tetrazolinone compounds having the general structure (V), as shown below.

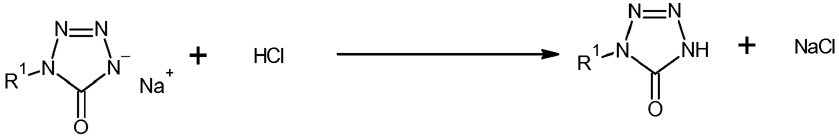

### EXAMPLE

The following example illustrates the invention without limiting it.

To 33 mL of dimethylformamide were added 0.64 g (2.5 mol%) of trimethylsilyl chloride and 15.5 g (1.03 equiv) of sodium azide. The mixture was stirred at 100 to 105°C and 27.6 g (1.0 equiv) of phenyl isocyanate were added over 45 min. The suspension was stirred for 1 h further at 100 to 105°C, cooled at 20°C, and slowly added to a mixture of 120 mL water and 2.0 g (5 mol%) of a sodium hydroxide solution, 25%. The solid by-products were removed by filtration. 3.8 mL (0.28 equiv) of an aqueous solution of sodium nitrite, 40% were added to the filtrate, followed by the addition of concentrated hydrochloric acid (36-37%) until the pH of the mixture was adjusted to <1. The product was then filtered and washed with 3x20 mL water. After drying at 50°C, 1-phenyl tetrazolinone was obtained (31.7 g) in 84% yield and with a purity of 99.4 area% (HPLC).

## Claims

1. A process for the preparation of a tetrazolinone compound, comprising a step of reacting an isocyanate compound with an azide salt in a solvent and in the presence of a silicon derivative as a catalyst which is substantially not consumed during the step of reacting the isocyanate compound with the azide salt, wherein the silicon derivative is prepared by reacting a silyl halide compound with an azide salt, wherein the tetrazolinone compound has the general formula (V): 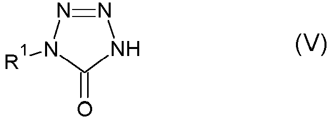
wherein R¹ is any carbon-containing group;
wherein the silicon derivative has the general formula (II): 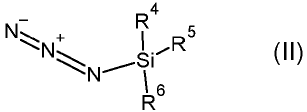
wherein each of R⁴, R⁵ and R⁶ are independently selected from aryl, alkyl, aralkyl and alkoxyalkyl groups; and wherein the silicon derivative is present in a molar amount of less than 0.5 equivalent relative to the isocyanate compound.

2. The process according to claim 1, wherein the azide salt is sodium azide.

3. The process according to claim 1 or 2, wherein the solvent is an amide, preferably selected from dimethylformamide, dimethylacetamide, N-methylpyrrolidone and combinations thereof.

4. The process according to any one of claims 1-3, wherein the silicon derivative is a trialkylsilyl azide, preferably trimethylsilyl azide.

5. The process according to any one of claims 1-4, wherein the silyl halide compound is a trialkylsilyl halide.

6. The process according to any one of claims 1-5, wherein the silyl halide compound is a silyl chloride compound.

7. The process according to any one of claims 1-6, wherein the silyl halide compound is trimethylsilyl chloride.

8. The process according to any one of claims 1-7, wherein the silicon derivative is employed in a molar amount of less than 0.1 equivalent, relative to the isocyanate compound.

9. The process according to any one of claims 1-8, wherein the isocyanate compound is an aryl isocyanate and the tetrazolinone compound is a 1-aryl-tetrazolinone.

10. The process according to any one of claims 1-9, wherein the step of reacting the isocyanate compound with the azide salt is performed at a temperature of at least 60°C, preferably of at least 80°C, and more preferably of at least 100°C.

11. The process according to any one of claims 1-10, wherein a tetrazolinone salt is obtained after the step of reacting the isocyanate compound with the azide salt; and the process further comprises an additional step of treating the tetrazolinone salt with an acid to obtain the tetrazolinone compound.

12. The process according to claim 11, comprising an intermediate purification step between the step of reacting the isocyanate compound with the azide salt and the additional step of treating the tetrazolinone salt with the acid.

13. The process according to claim 11 or 12, further comprising a step of treating the tetrazolinone salt with sodium nitrite, before the additional step of treating the tetrazolinone salt with the acid, and preferably after the intermediate purification step.

## Patentansprüche

1. Verfahren zur Herstellung einer Tetrazolinonverbindung, umfassend einen Schritt des Umsetzens einer Isocyanatverbindung mit einem Azidsalz in einem Lösungsmittel und in Gegenwart eines Siliciumderivats als Katalysator, das während des Schritts des Umsetzens der Isocyanatverbindung mit dem Azidsalz im Wesentlichen nicht verbraucht wird, wobei das Siliciumderivat hergestellt wird durch Umsetzen einer Silylhalogenidverbindung mit einem Azidsalz, wobei die Tetrazolinonverbindung die allgemeine Formel (V) aufweist: 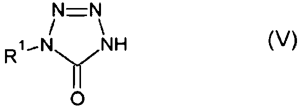
wobei R¹ eine beliebige kohlenstoffhaltige Gruppe ist;
wobei das Siliciumderivat die allgemeine Formel (II) aufweist: 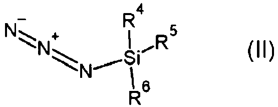
wobei jedes von R⁴, R⁵ und R⁶ unabhängig voneinander aus Aryl-, Alkyl-, Aralkyl- und Alkoxyalkylgruppen ausgewählt ist; und
wobei das Siliciumderivat in einer molaren Menge von weniger als 0,5 Äquivalent relativ zu der Isocyanatverbindung vorliegt.

2. Verfahren nach Anspruch 1, wobei das Azidsalz Natriumazid ist.

3. Verfahren nach Anspruch 1 oder 2, wobei das Lösungsmittel ein Amid ist, vorzugsweise ausgewählt aus Dimethylformamid, Dimethylacetamid, N-Methylpyrrolidon und Kombinationen davon.

4. Verfahren nach einem der Ansprüche 1 bis 3, wobei das Siliciumderivat ein Trialkylsilylazid ist, vorzugsweise Trimethylsilylazid.

5. Verfahren nach einem der Ansprüche 1 bis 4, wobei die Silylhalogenidverbindung ein Trialkylsilylhalogenid ist.

6. Verfahren nach einem der Ansprüche 1 bis 5, wobei die Silylhalogenidverbindung eine Silylchloridverbindung ist.

7. Verfahren nach einem der Ansprüche 1 bis 6, wobei die Silylhalogenidverbindung Trimethylsilylchlorid ist.

8. Verfahren nach einem der Ansprüche 1 bis 7, wobei das Siliciumderivat in einer molaren Menge von weniger als 0,1 Äquivalent relativ zu der Isocyanatverbindung eingesetzt wird.

9. Verfahren nach einem der Ansprüche 1 bis 8, wobei die Isocyanatverbindung ein Arylisocyanat ist und die Tetrazolinonverbindung ein 1-Aryl-Tetrazolinon ist.

10. Verfahren nach einem der Ansprüche 1 bis 9, wobei der Schritt des Umsetzens der Isocyanatverbindung mit dem Azidsalz bei einer Temperatur von mindestens 60°C, vorzugsweise von mindestens 80°C und stärker bevorzugt von mindestens 100°C durchgeführt wird.

11. Verfahren nach einem der Ansprüche 1 bis 10, wobei nach dem Schritt des Umsetzens der Isocyanatverbindung mit dem Azidsalz ein Tetrazolinonsalz erhalten wird; und das Verfahren ferner einen zusätzlichen Schritt des Behandelns des Tetrazolinonsalzes mit einer Säure umfasst, um die Tetrazolinonverbindung zu erhalten.

12. Verfahren nach Anspruch 11, umfassend einen zwischen dem Schritt des Umsetzens der Isocyanatverbindung mit dem Azidsalz und dem zusätzlichen Schritt des Behandelns des Tetrazolinonsalzes mit der Säure liegenden Zwischenreinigungsschritt.

13. Verfahren nach Anspruch 11 oder 12, ferner umfassend einen Schritt des Behandelns des Tetrazolinonsalzes mit Natriumnitrit vor dem zusätzlichen Schritt des Behandelns des Tetrazolinonsalzes mit der Säure und vorzugsweise nach dem Zwischenreinigungsschritt.

## Revendications

1. Procédé de préparation d'un composé de tétrazolinone, comprenant une étape consistant à faire réagir un composé isocyanate avec un sel azoture dans un solvant et en présence d'un dérivé de silicium en tant que catalyseur qui n'est sensiblement pas consommé pendant l'étape de réaction du composé isocyanate avec le sel azoture, dans lequel le dérivé du silicium est préparé en faisant réagir un composé halogénure de silyle avec un sel azoture, dans lequel le composé de tétrazolinone a la formule générale (V) : 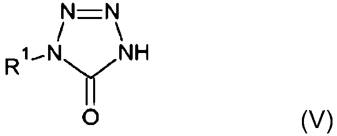 où R¹ est un quelconque groupe contenant du carbone ;
dans lequel le dérivé de silicium a la formule générale (II): 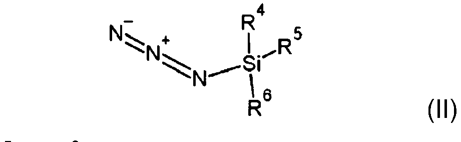 où chacun de R⁴, R⁵ et R⁶ est indépendamment choisi parmi des groupes aryle, alkyle, aralkyle et alcoxyalkyle ; et dans lequel le dérivé de silicium est présent en une quantité molaire inférieure à 0,5 équivalent par rapport au composé isocyanate.

2. Procédé selon la revendication 1, dans lequel le sel azoture est l'azoture de sodium.

3. Procédé selon la revendication 1 ou la revendication 2, dans lequel le solvant est un amide, de préférence choisi parmi le diméthylformamide, le diméthyl-acétamide, la N-méthylpyrrolidone et des combinaisons de ceux-ci.

4. Procédé selon l'une quelconque des revendications 1 à 3, dans lequel le dérivé de silicium est un azoture de trialkylsilyle, de préférence l'azoture de triméthylsilyle.

5. Procédé selon l'une quelconque des revendications 1 à 4, dans lequel le composé halogénure de silyle est un halogénure de trialkylsilyle.

6. Procédé selon l'une quelconque des revendications 1 à 5, dans lequel le composé halogénure de silyle est un composé chlorure de silyle.

7. Procédé selon l'une quelconque des revendications 1 à 6, dans lequel le composé halogénure de silyle est le chlorure de triméthylsilyle.

8. Procédé selon l'une quelconque des revendications 1 à 7, dans lequel le dérivé de silicium est employé en une quantité molaire inférieure à 0,1 équivalent, par rapport au composé isocyanate.

9. Procédé selon l'une quelconque des revendications 1 à 8, dans lequel le composé isocyanate est un isocyanate d'aryle et le composé de tétrazolinone est une 1-aryltétrazolinone.

10. Procédé selon l'une quelconque des revendications 1 à 9, dans lequel l'étape de réaction du composé isocyanate avec le sel azoture se fait à une température d'au moins 60 °C, de préférence au moins 80 °C et plus préférentiellement au moins 100 °C.

11. Procédé selon l'une quelconque des revendications 1 à 10, dans lequel un sel de tétrazolinone est obtenu après l'étape de réaction du composé isocyanate avec le sel azoture ; et le procédé comprend en outre une étape supplémentaire de traitement du sel de tétrazolinone avec un acide afin d'obtenir le composé de tétrazolinone.

12. Procédé selon la revendication 11, comprenant une étape intermédiaire de purification entre l'étape de réaction du composé isocyanate avec le sel azoture et l'étape supplémentaire de traitement du sel de tétrazolinone avec l'acide.

13. Procédé selon la revendication 11 ou la revendication 12, comprenant en outre une étape de traitement du sel de tétrazolinone avec du nitrite de sodium, avant l'étape supplémentaire de traitement du sel de tétrazolinone avec l'acide, et de préférence après l'étape intermédiaire de purification.
